# EUROPEAN PATENT APPLICATION

(11) **EP 4 810 614 A1**
(43) Date of publication of application: **23.09.2026**
(21) Application number: 24889961.9
(22) Date of filing: 29.03.2024
(51) Int. Cl.: C12N 15/70, C07K 14/62

(54) **SYNTHETIC ORGANELLE-ASSISTED GENE EXPRESSION METHOD, DNA CONSTRUCT, EXPRESSION SYSTEM, AND USE OF SYNTHETIC ORGANELLE**

(30) Priority: 13.11.2023 CN 202311506498
(71) Applicant: Ailurus Ltd, Edinburgh, EH6 7BD (GB)
(72) Inventor: GUO, Haotian, London (GB)
(74) Representative: Finnegan Europe LLP
(86) International application number: PCT/CN2024/085047
(87) International publication number: WO 2025/102587

(57) **Abstract**

The present disclosure relates to a synthetic organelle-assisted gene expression method, a DNA construct, an expression system, and uses thereof. The present disclosure provides an efficient and feasible method for eliminating or reducing stress and/or toxicity of gene expression products on the host, and for providing a suitable and controllable maturation environment for gene expression products, thereby optimizing gene expression.

## Description

### Technical Field

The present disclosure relates to the field of engineering, and in particular to a synthetic organelle-assisted gene expression method, a DNA construct, an expression system, and uses thereof.

### Background Art

Gene expression generally refers to the process of synthesizing and expressing a target gene in a host. Hosts commonly used include various cellular expression systems, such as bacterial expression systems (e.g. *E. coli* and *Bacillus subtilis*), yeast expression systems, and animal cell expression systems (e.g. insect cells and human cells). The host may also be a cell-free expression system, such as a cell lysate. This process plays an important role in biotechnology, molecular biology, and drug development because it makes it possible to prepare a large amount of a target product, in particular a recombinant protein of interest, for research, therapy, or industrial applications.

However, the interaction between the expression process and the host gives rise to a series of complex technical and biological challenges.
a. Energy and material burden imposed by the expression process: when an exogenous gene is to be expressed, the host must allocate energy and resources to meet the expression demand. This increases the burden on the cells and may limit the normal growth and health status of the cells.
b. Stress on host caused by expression products: this makes gene expression more difficult because a balance must be found between normal host activity and protein expression. In particular, when the host is a cellular expression system, cell growth may be inhibited or even stopped. According to the mechanism, the source of stress on a host includes:
   a) Exogenous products occupy the space within the host, thereby non-specifically interfering with normal physiological processes, and causing general stress.
   b) Some products have specific toxicity and can damage the host even at very low expression levels. For example, when the target product is a nuclease, it may cleave and degrade host DNA or RNA; when the target product is an exogenous tRNA, it may lead to translation errors.
c. Expression products may fail to mature in the host's intracellular environment. Maturation of a target product requires specific physicochemical conditions and biochemical reactions that may be absent in the host, so that the target product may not mature correctly.

For example:
a) When an exogenous protein is overexpressed, misfolding often occurs. Such misfolded proteins may lack normal biological activity and tend to aggregate into insoluble inclusion bodies.
b) When a protein containing disulfide bonds is expressed in *E. coli*, disulfide bonds cannot form because of the reducing cytoplasmic environment in *E. coli*, and the protein therefore may not fold correctly.
c) Some target products require post-processing to attain the correct activity, such as zymogen cleavage, antibody glycosylation, intron removal from RNA, or RNA editing.

These difficulties are common challenges in gene expression, and researchers have long explored a variety of methods and strategies to overcome them so as to achieve efficient and controllable expression.

The present disclosure aims to address challenge b (stress on host) and challenge c (product maturation), and to provide an innovative method for improving expression and creating new possibilities for research and application in relevant fields.

More specifically, the present disclosure addresses two major challenges: stress on host caused by expression products, for example by expression of toxic proteins, and inability of expression products to mature in the host's internal environment, for example protein misfolding. These challenges often arise in biotechnology and molecular biology and limit the efficiency and feasibility of expression. Some related situations and prior-art approaches are set out below. These approaches typically require complicated work and are not always successful, and no relatively universal solution is currently available.
a. Selecting a suitable host system: the most suitable host expression system is selected according to the characteristics of a target gene. For example, a cell-free system is used to express cytotoxic proteins, and a human cell line is used to express human antibodies. Proteins containing disulfide bonds may be expressed in mutant *E. coli* strains such as SHuffle, in which reducing enzymes are deleted. However, many host systems are expensive to use and complex to operate. Moreover, such methods are suitable only for single-gene expression or for several genes having similar properties, and not extendable to the expression of more complex DNA constructs.
b. Expression vector engineering: for example, selecting an appropriate promoter, adjusting expression strength, using an enhancer, or the like in order to adjust the expression level. However, these methods cannot satisfy multiple objectives simultaneously. For example, a weak promoter may avoid excessive expression and thus reduce inclusion-body formation, but cannot deliver an efficient high-level expression of an exogenous protein. A strong promoter, on the other hand, increases cellular burden and thus limits efficient expression of a target protein.
c. Optimization of expression conditions: the expression level can be influenced by controlling of cell-culture conditions such as temperature, pH, and oxygen supply, use of inducible expression systems, and adjustment of the induction conditions. For example, for proteins prone to misfolding and aggregation, low-temperature expression may promote proper folding. In some cases, however, such optimizations may not effectively improve expression.
d. Metabolic engineering: altering the metabolic pathways of a host may increase the yield of a target product.
e. Protein engineering: for toxic proteins or proteins prone to formation of inclusion bodies, methods have been proposed such as changing the protein structure, adjusting the expression rate, using specific tags or split proteins, in order to reduce adverse effects on the cell. These methods, however, may also reduce expression efficiency.
f. Post-processing: inactive target products produced by a host may be purified and then renatured, cleaved, or modified in order to restore the correct structure. Post-processing, however, is a highly complex and expensive process, and is not suitable for many target products.

At present, no universal solution can simultaneously achieve high expression, low stress/toxicity, correct folding, and post-expression modifications.

Prior Art Document 1 relates to optimization of expression vectors and expression conditions.

The technical solution of Prior Art Document 1 is generally to optimize expression by engineering the expression vector and the expression conditions.

For expression vectors, appropriate promoters, expression vectors, and regulatory elements are selected in order to control and optimize gene expression. This may be achieved, for example, by adjusting the copy number or promoter activity in the expression vector.

For expression conditions, culture conditions such as the temperature, pH, oxygen supply, and medium composition are adjusted; for inducible vectors, induction conditions are also adjusted. By changing the expression conditions, expression efficiency may be improved, and protein folding and stability may also be improved.

Prior Art Document 1 has the following drawback: although it can optimize expression to some extent, it lacks clear theoretical guidance, requires substantial trial and error for different target genes, and is not always effective.

Prior Art Document 2 relates to optimization of the expression host.

The technical solution of Prior Art Document 2 is as follows.

A suitable host is selected for expression in view of the characteristics of a target gene, and corresponding post-processing steps are then designed for that host.

For example, a human cell line or insect cells are used to express animal-derived proteins. Optimization of the host's metabolic network and the transcription and translation system can further improve expression. For example, an engineered CHO cell line was used for high-level antibody expression and production, and the SHuffle strain obtained by altering the reducing environment within *E. coli* was used for production of proteins containing disulfide bonds.

As another example, a cell-free expression system is used as an alternative in order to address difficulties encountered in cellular expression. Cell-free expression systems generally involve the following key steps.
1) Cell lysis and preparation of cell extract: cytoplasmic and nucleic-acid materials are extracted from cells to obtain an extract containing the basic biomolecular machinery of the cells.
2) Addition of exogenous DNA or RNA: a DNA or RNA encoding a target protein, typically synthesized by a synthetic-biology method, is added to the extract.
3) Translation and protein synthesis: in an *in vitro* environment, the cellular machinery present in the extract, such as ribosomes, tRNA, and amino acids, is used to translate and synthesize the target protein.
4) Protein purification and extraction: the target protein is purified and extracted from the reaction system, typically by affinity chromatography, gel electrophoresis, or other separation techniques.
5) Analysis and confirmation: the protein is analyzed and confirmed to ensure that its structure and function meet expectations.

Prior Art Document 2 has the following drawbacks.
a) Great operational complexity: transformation and transfection steps vary significantly among different hosts, the learning difficulty is considerable, and it is difficult to genetically manipulate and culture some hosts.
b) High Cost: some host systems are expensive to use. Mammalian cell lines, for example, require costly serum-containing media. Cell-free systems often require expensive exogenous factors, such as energy sources, amino acids, ions, and protein factors, to support protein synthesis.
c) A given host may possess some desirable properties but lack others. For example, a cell-free system is not affected by cellular toxicity, but lacks post-translational modification and generally cannot provide glycosylation, phosphorylation, and other modifications at the same level as a living cell, which may affect protein functionality and stability. In cell-free systems, the protein expression level may also be relatively unstable and influenced by reaction conditions and component concentrations. For example, the SHuffle strain may promote disulfide-bond formation, but its own physiological activity and stress tolerance may be accordingly reduced.
d) A single host is generally suitable only for a series of genes having similar properties, and it is difficult to extend its applicability to multigene co-expression and expression of more complex DNA constructs.

CN112575016A discloses "Construction of Membraneless Organelles in Prokaryotes and Use thereof", which successfully constructed membraneless compartments in *E. coli* using recombinant spider fibroin and arthropod elastin-like protein, and achieved intracellular co-localization of target functional proteins by fusing spider fibroin or arthropod elastin-like protein to different cargo proteins through DNA recombination technology, thereby constructing membraneless compartments with biological activity.

WO2023015190A1 discloses a method for controlling cellular processes in mammalian cells through synthetic organelles, which uses an arginine/glycine-rich (RGG) protein domain capable of liquid-liquid phase separation (LLPS) connected with a high-affinity coiled-coil tag to enrich endogenous proteins also connected with a coiled-coil tag, thereby controlling cell behavior by isolating or releasing endogenous proteins.

However, neither of the above applications involves the possibility that the synthetic organelles can assist the proper folding of the target gene products or target proteins, and the existing data do not support such a conclusion. In fact, both applications clearly describe that the target protein itself can be correctly expressed and perform its function. CN112575016A describes proteins having biological activity, and WO2023015190A1 describes endogenous proteins capable of regulating cellular functions. In addition, in CN112575016A the target protein is connected to spider fibroin and arthropod elastin-like protein by a fusion strategy, so the folding and maturation of the target protein may be interfered with by the protein-aggregation process and it is difficult to measure and control these properties. In WO2023015190A1, the host cell is a mammalian cell, and proteins expressed in mammalian cells generally receive appropriate modifications and are therefore directly functional. The drawbacks of that approach are that it is technically difficult to operate, time consuming, and uneconomical.

Prokaryotic expression systems such as *E. coli* are currently the most widely used expression systems because culture scale-up is simple, rapid, economical, and suitable for large-scale manufacture. Their drawbacks are that expressed eukaryotic proteins may fail to fold properly or fail to undergo glycosylation, expressed proteins often form insoluble inclusion bodies that require complex renaturing treatment to become active, and it is difficult to obtain a large amount of soluble protein in an *E. coli* expression system.

In summary, although a number of methods have been proposed to optimize expression, the present disclosure aims to provide a comprehensive and more universal solution capable of addressing the aforementioned challenges at the same time. This innovation will help promote research and application in related fields and open up new possibilities for broader scientific and engineering use.

### Summary of the present disclosure

The principal object of the present disclosure is to solve two key technical problems in cellular expression, namely, eliminating or reducing stress and/or toxicity caused by gene expression products to a host, and providing a suitable and controllable maturation environment for gene expression products, thereby optimizing gene expression.

Expression products may cause stress or even toxicity to the physiological activities of the host. The present disclosure therefore aims to separate the expression products from the host's native physiological activities so as to decouple the effects by exogenous expression from endogenous physiology. More specifically, expression products occupy space within the host and non-specifically interfere with normal physiological processes, thereby generating general stress and adversely affecting normal physiology of the host. The present disclosure aims to provide a method that can significantly increase gene expression while reducing stress on host so as to achieve efficient expression. Certain products also have specific toxicity and can damage the host even at low expression levels. In particular, when the host is a cellular system, expression of such genes often adversely affects the cells and may even cause cell death. The present disclosure aims to provide a method that can optimize expression of toxic products, mitigate adverse effects on the host, and at the same time maintain a high expression level.

The host's internal environment may cause incorrect maturation of expression products. The maturation process of a target product requires specific physicochemical conditions and biochemical reactions that may be unavailable in the host, leading to incorrect maturation of the target product and production of inactive expression products. For example, during expression of exogenous proteins, especially at a high expression level, the proteins often fold into incorrect three-dimensional structures inside the host, thereby losing normal biological activity and forming insoluble aggregates. For example, in *E. coli*, because the reducing environment in the cytoplasm makes it impossible to stably form disulfide bonds in a protein, proinsulin produced in *E. coli* generally exists as misfolded inclusion bodies. The present disclosure aims to provide a method that provides the expression product with a maturation environment decoupled from the host's internal environment, so as to reduce incorrect maturation of the expression product without disrupting the host's native physiology, thereby efficiently obtaining active target products.

Overall, the present disclosure aims to provide a comprehensive and efficient method capable of simultaneously solving the key technical problems associated with stress on host and product maturation. By solving these problems, the present disclosure seeks to provide a more effective and more feasible solution for research and applications in bioengineering, drug development, protein production, and other related fields, thereby promoting scientific and technological progress.

To achieve the above purposes, the present disclosure provides the following technical solutions.

According to the present disclosure, a synthetic organelle is constructed in a host so that after a target gene is expressed the expression product can enter the synthetic organelle in a timely manner, thereby achieving the following effects.
1. Elimination of stress on and/or toxicity to the host: by separating the expression product from the host, the probability of contact between the product and the other molecules within the host is reduced, which not only minimizes the impact by the product on the host to the greatest extent possible, but also can change the kinetic balance and feedback inhibition of the gene expression by lowering the concentration of the product in the host, thereby promoting generation of the expression product.
2. Provision of a controllable and suitable maturation environment for the expression product: the synthetic organelle provides an orthogonal environment different from the host's internal environment and allowing programmed regulation; the expression product enriched in the synthetic organelle can complete correct folding, modification, cleavage, and other maturation processes.

In one aspect, the present disclosure provides a synthetic organelle-assisted gene expression method, comprising the following steps:
a. constructing a synthetic organelle in a host; and
b. co-expressing the synthetic organelle and a gene expression product so that the gene expression product can be localized in the synthetic organelle.

In one embodiment, the host includes a cell-free expression system and a cellular host, preferably a prokaryotic cellular host, more preferably *E. coli*.

In one embodiment, the synthetic organelle is an artificially designed compartmentalized structure. Preferably, the synthetic organelle is a membraneless organelle, namely a biomolecular condensate formed in the host from polypeptides and/or proteins and/or DNA and/or RNA and/or nucleoprotein complexes. Preferably, the biomacromolecules used to construct the synthetic organelle are nucleotide sequences with 10-1000 consecutive repeats of nucleotide repeat units, such as CAG triplets, CCUG, GGGAA, GGGGCC, etc.; amino acid sequences with 3-500 consecutive repeats, such as repeats of resilin-like peptides (RLP); intrinsically disordered regions, such as FUS protein, ParB protein; polymerization of multivalent biomacromolecules, such as PTB protein with UCUCU sequence repeats, artificially designed binary protein two-dimensional structures, and artificially designed binary RNA two-dimensional structures.

In one embodiment, the gene expression product is an RNA, a protein, a nucleoprotein complex, or a small molecule synthesized by a heterologous encoding DNA. Preferably, the product is a recombinant protein, such as superfolder green fluorescent protein, restriction endonuclease DpnI, restriction endonuclease BsaI, human proinsulin, or ROR-alpha. Preferably, the gene expression product matures in the synthetic organelle.

In one embodiment, in step b, the gene expression product is localized in the synthetic organelle by non-specific and/or specific binding. Preferably, a specific receptor/ligand pair is used, the synthetic organelle has the receptor, and the gene expression product is connected with the ligand, such that the synthetic organelle can specifically bind the gene expression product. Preferably, the receptor/ligand pair includes complementary nucleic acid molecule pairs; capsid proteins of RNA phages such as MS2, PP7, and Qbeta and translation operator RNA hairpins; two segments of split proteins, such as split green fluorescent protein, alpha and omega fragments of beta-galactosidase, split T7 RNA polymerase.

In another aspect, the present disclosure provides a DNA construct comprising nucleotide sequence 1 that guides the host to construct the synthetic organelle defined above, and/or nucleotide sequence 2 that guides and regulates the generation of the gene expression product, preferably, wherein the DNA construct is composed of said nucleotide sequences 1 and 2 and a plasmid vector, and said plasmid vector is for example pACYC184, pACYC177, pET28a(+), pET28b(+), pET-5a(+), pET43.1a, pET-37b(+), pCDFDuet-1, pCOLADuet-1, pRSFDuet-1, pETDuet-1, pUC57, pUC19, pBAD, pBluescript II SK(+), pTrcHis C, pTrcHis A, pTrcHis2C, pBV221, pQE-70, pCold III, pRSET-CFP, pRSET-BFP, pGFPuv, pKD46, pKD4, pTYB1, PinPoint Xa-2, pTWIN1, pRSET C, pSB1C3, pSB3C5, pSB4C5, or pSB4K5. Preferably, the upstream promoter is a constitutive promoter or an inducible promoter.

In yet another aspect, the present disclosure provides an expression system comprising the above DNA construct. Preferably, the expression system refers to a system that simultaneously constructs the synthetic organelle and generates the gene expression product in a prokaryotic cell, and more preferably, the expression system is an *E. coli* expression system.

In a further aspect, the present disclosure provides use of a synthetic organelle in eliminating stress and/or toxicity caused by gene expression to the host and/or in decoupling a gene expression product from the host's internal environment, comprising co-expression of the synthetic organelle and the gene expression product in the host so that the gene expression product is localized in the synthetic organelle.

In one embodiment, the use comprises improving the physiological activity and tolerance, the biomass under the same conditions, the gene expression rate, and/or the cell growth rate of the host.

In another embodiment, the above method or use comprises promoting maturation of the gene expression product. Preferably, the maturation comprises one or more of: folding of biomacromolecules; post-expression modifications such as disulfide-bond formation, glycosylation, methylation, and acetylation; cleavage and ligation, such as zymogen cleavage activation, RNA splicing, intein-mediated protein cleavage, and self-cyclization; and non-covalent assembly, such as dimerization and polymerization.

### Beneficial Effects

The technical solution according to the present disclosure provides multiple beneficial effects directed to solving the problems existing in the prior art, and directed to providing a more efficient and more feasible method for eliminating stress and/or toxicity caused by expression products to the host and for providing the expression product with a suitable and controllable maturation environment, thereby optimizing the gene expression. These beneficial effects include the following.
(1) High yield: the technical solution according to the present disclosure can improve the expression level without increasing the burden on the host. This makes it possible to obtain a higher yield of expression product so as to satisfy the need for high expression in industrial and research applications.
(2) Reduced adverse effects of toxic products: the method according to the present disclosure can optimize expression of toxic products and reduce their adverse effects on the host. This can improve the health status and reduce mortality of the host, and facilitate production of more expression products that are toxic to the host, which is important in drug development and bioengineering.
(3) Reduced misfolding: the method according to the present disclosure can avoid misfolding of exogenous proteins in the cytoplasm and reduce generation of insoluble materials such as inclusion bodies.
(4) Provision of an environment allowing for correct modifications: the method according to the present disclosure can provide a suitable environment for post-expression modifications of the products, for example disulfide-bond formation.
(5) Consistent high-level expression: the technical solution according to the present disclosure can provide a more consistent protein-expression performance and reduce variations between batches, thereby supporting stable experimental results and reproducible production processes.
(6) Resource efficiency: the method according to the present disclosure can improve expression efficiency and lower research cost and production cost by reducing waste of energy and cellular resources.
(7) Broad applicability: unlike certain prior-art methods that are suitable only for specific types of proteins or cell systems, the technical solution according to the present disclosure has broad applicability and can be used for various types of proteins and cells.

In summary, the technical solution according to the present disclosure is intended to provide a comprehensive and efficient method for solving key problems in cellular expression and for providing a more feasible and more efficient solution for scientific research, biopharmaceutical applications, and other related fields. These beneficial effects will promote technical development and create new opportunities for innovation and progress in the life sciences.

### Brief Description of the Drawings

Figure 1 shows the growth curves of *E. coli* under three expression conditions. The blue curve represents the growth curve when no GFP was expressed, the green curve represents the growth curve when only tdMCP-GFP was induced and expressed, and the red curve represents the growth curve when tdMCP-GFP and the rCAG organelle were co-expressed. The vertical bars show the standard deviation, the x-axis shows growth time, and the y-axis shows absorbance at 600 nm.
Figure 2 shows a gel electrophoresis image when the target protein was DpnI. M denotes a protein marker; P denotes an insoluble precipitated product; S denotes the purified soluble protein product; and the red asterisk marks the DpnI protein (30 kDa).
Figure 3 shows a gel electrophoresis image when the target protein was BsaI. M denotes a protein marker; P denotes an insoluble precipitated product; S denotes the purified soluble protein product; the red asterisk marks the BsaI protein (about 60 kDa); and the blue asterisk marks tdMCP-BsaI before cleavage.
Figure 4 shows a gel electrophoresis image when the target protein was proinsulin. M denotes a protein marker; P denotes an insoluble precipitated product; S denotes the purified soluble protein product; and the red asterisk marks the proinsulin protein (12 kDa).
Figure 5 shows a gel electrophoresis image when the target protein was ROR-alpha. M denotes a protein marker; S denotes the purified soluble protein product; and the red asterisk marks the ROR-alpha protein (about 60 kDa).

### Detailed Description of the Embodiments

The present disclosure is described in more details below.

### 1. Synthetic organelles

Cells generally have a complex spatial organization, one of which is called compartmentalization, referring to spatially organized structures formed within a cell to perform specific functions and biochemical reactions. Such compartmentalized structures are also often called organelles, which are to cells what organs are to individuals.

Organelles are either separately enclosed in their own lipid bilayers, called membrane-bound organelles, such as mitochondria, chloroplasts, etc.; or are spatially independent functional units without lipid bilayers around them, known as membraneless organelles, which are also compartments visible under optical microscopes. In 2009, Hyman and Brangwynne discovered and revealed that membraneless organelles are formed by biomacromolecules in cells through phase separation (1. C. P. Brangwynne et al., Germline P granules are liquid droplets that localize by controlled dissolution/condensation. Science 324, 1729-1732 (2009)).

Synthetic organelles are an innovative bioengineering concept, also called "organelle-like structures", "artificially synthesized organelles", or "artificial organelles". Synthetic organelles aim at designing and construction of small functional units similar to natural organelles for achieving compartmentalization in cells (or *in vitro* test tube environments), and are applied to control biosynthesis, metabolic pathways, or other biological processes. The construction of synthetic organelles involves introducing exogenous DNA constructs into host cells. These exogenous DNAs include exogenous coding genes, non-coding DNA, regulatory elements, etc., for guiding cell-free expression systems or host cells to synthesize specific polypeptides, proteins, DNA, RNA, enzymes, or other biomolecules. Some of these molecules serve to construct the synthetic organelle structure, while other molecules perform the preset functions of synthetic organelles, such as biocatalysis, receptor recognition, protein sorting, etc. Synthetic organelles can be constructed through heterologous multimer self-assembly of proteins, DNA, and RNA, or through modification of cell membrane structures. Since the phase separation mechanism for membraneless organelles was revealed, synthetic organelles can also be constructed by assembly of biomacromolecules capable of phase separation.

The present disclosure provides a method for decoupling a gene expression product from the host's internal environment by using a synthetic organelle. The synthetic organelle can be constructed substantially from polypeptides and/or proteins and/or DNAs and/or RNAs and/or nucleoprotein complexes. For example, repeated triplet ribonucleic acids (CAG)n spontaneously assemble into RNA condensates/droplets through liquid-liquid phase separation when transcribed in a cell. The synthetic organelle can also contain other substances according to the characteristics of a target product. For example, expressing P9 and P12 proteins of φ6 phage can recruit lipid molecules to form membrane structures.

The biomacromolecular components for constructing a synthetic membraneless organelle may be selected from the group consisting of: nucleotide sequences with 10-1,000 consecutive repeats of repeat units, such as CAG triplets, CCUG, GGGAA, GGGGCC, etc.; amino acid sequences with 3-500 consecutive repeats, such as repeats of resilin-like peptides (RLP); intrinsically disordered regions, such as FUS protein and ParB protein; polymerization of multivalent biomacromolecules, such as PTB protein with UCUCU sequence repeats; artificially designed binary protein two-dimensional structures; and artificially designed binary RNA two-dimensional structures.

Certainly, the synthetic organelle according to the present disclosure can also be constructed from polypeptides and/or proteins and/or DNAs and/or RNAs and/or nucleoprotein complexes of other sequences, for example:
- RNA:
   ∘ Repeated triplet ribonucleic acids, such as (CAG)n, (GGU)n, (AUG)n, (CGG)n, (GUU)n, (CGU)n, (AUU)n, (ACG)n, (AAU)n, (AUC)n, (CCG)n, (AGC)n, (AGU)n, (ACU)n, where n is 10-1,000;
   ∘ Repeated quadruplet ribonucleic acids, such as (CCUG)n, where n is 10-1,000;
   ∘ Repeated quintuplet ribonucleic acids, such as (GGGAA)n, where n is 10-1,000;
   ∘ Repeated sextuplet ribonucleic acids, such as GGGGCC(rGGGGCC), i.e., (GGGGCC)n, (AGGGCC)n, (GGAGCC)n, (GGGGAC)n, (AGGACC)n, where n is 10-1,000;
   ∘ Unary synthetic RNA scaffolds, for example ("**XX--XX**" in bold represents sites where functio nal nucleic acid aptamers or ribozymes can be inserted): GGGTAGGCGCCTAGCCTAATGTACATTA AGTTATTTTTCCGGATGAATAGAATATATTCTAATAACGCAGG**XX--XX**CCTCGAATACGAGC TGGG**XX--XX**CCCAGGAAGTGTTCGCACTTCTCTCGTATTCGATTGCGACTAGT;
   ∘ Binary synthetic RNA scaffolds, for example ("**XX--XX**" in bold represents sites where functional nucleic acid aptamers or ribozymes can be inserted): d2-1 GGGTCAGGAATCCTCCTGATAGCTATTTGGACAATTACGTACGTAGTTGATGACAACTACAT GAAAATAAGGG**XX--XX**CCCTCTAGA and d2-2 GGGTAGTTGTTATGGATTCCTGATTTATGGG**XX--XX**CCACTAGT;
- DNA:
   ∘ ssDNA sequences identical to the above RNA sequences;
   ∘ ssDNA capable of forming nanopolymers, such as nanopolymers of the following four ssDNAs ("**XX--XX**" in bold represents sites where functional nucleic acid aptamers or ribozymes can be inserted): oligo-1 TGCGCAATCCC**XX--XX**CTTGAGCACGCCAACATCACCGTAATT, oligo-2 GCGGCTAGCAGAGCATTCGGGA**XX--XX**GACTATGGCTGTATTT, oligo-3 GGCGTGCTCAC**XX--XX**TCGGATTGCGCATGCTAGCCGCTATTT, oligo-4 CGGTGATGTTCAGCCATAGTAG**XX-XX**GCCGAATGCTCTATTT, etc.;
- Proteins:
   ∘ Self-assembled protein nanoparticles and nanostructures, such as: Carboxysome shell proteins; Metabolosome shell proteins; Bacterial microcompartment (BMC) shell proteins BMC-H, BMC-T, and BMC-P; *Haliangium ochraceum* bacterial microcompartment capsid protein T1 (HO BMC Shell T1); *Thermotoga maritima* encapsulation protein EncTm; Mixed phage Qβ virus-like particles; Ferritin; artificially synthesized protein nanocages, such as antibody Fc nanocages; artificially synthesized protein self-assembled fibers, such as CC-Di-B-PduA protein; artificially synthesized protein self-assembled two-dimensional structures; and artificially synthesized protein self-assembled three-dimensional structures, such as ATC-HL3 protein;
   ∘ Intrinsically disordered proteins (IDP), such as: Resilin-like peptides (RLP); Elastin-like peptides (ELP); RNA helicase LAF-1 RGG domain repeats; RNA helicase DDX4; *Caulobacter crescentus* ribonuclease E (RNaseE); IDP homologous protein sequences (GRGDSPYS)n, and mutants (GRGDSPYSGRGDSPYSGRGDSPYSGRGDSPVS)n, (GRGDSPYSGRGDSPVS)n, where n is 3-500; ParB protein; FUS protein; IbpA protein; PopZ protein; PodJ protein; conserved nuclear pole protein fiber protein FIB-1;
   ∘ Protein amyloid precipitates, such as: AEAEAKAKAEAEAKAK, LELELKLKLELELKLK, human β-amyloid peptide Ab42 (F19D);
   ∘ Other proteins that cause aggregation, such as: *E. coli* beta-galactosidase; Maltose-binding protein mutant MalE31; *Clostridium cellulovorans* cellulose-binding domain (CBD); *Clostridium thermocellum* endoglucanase D (EGD); fusion proteins of the following proteins from *Brucella abortus* and fluorescent proteins: PdhS-mCherry, FumC-YFP, and DivK-YFP; fusion proteins of the following proteins from *E. coli* and fluorescent proteins: ClpX-sfGFP, ClpP-sfGFP, ClpP-mCherry, ClpX-mCherry, ClpX-mCherry2, ClpX-Venus, LacZ-Dronpa, TetR-Dronpa, P22C2-Dronpa, HKCI-Dronpa; Tandem repeat SH3 (polySH3) with tandem repeat PRM (polyPRM); Tandem repeat SIM (polySIM) with tandem repeat SUMO (polySUMO); and Pentameric nuclear pole protein Npm1, etc.;
- Nucleoprotein multimer complexes:
   ∘ Complex of long non-coding RNA (lncRNA) and RNA-binding protein, wherein the nucleic acid scaffold include for example mammalian nuclear paraspeckle assembly transcript 1 isoform 2 in paraspeckles; intergenic spacer lncRNA in amyloid bodies; human satellite III (SatIII) lncRNA in nuclear stress bodies; Drosophila heat shock RNA (Hsr) ω; Schizosaccharomyces meiRNA; RNA-binding proteins such as PTB protein; FUS protein; and Histone BRD4;
   ∘ Polypeptide sequences and polynucleotides, such as polypeptide RRASLRRASL with polyuridylic acid (polyU) having a molecular weight of 600-1,000 kDa; synthetic polypeptides RP3, SR8, and polyU, etc.

Synthetic organelles may be constructed entirely from biomolecules synthesized by the host under the guidance of an exogenous DNA construct, may be constructed from partial exogenous molecules combined with endogenous molecules of the host, or may be obtained by modification of original endogenous organelles or cytoskeleton structures of the host. For example, in a method for codon expansion using synthetic organelles disclosed in EP 19157257.7, the synthetic organelle is formed by exogenous PylRS::FUS::KIF16B1-400 fusion protein, MCP::EWSR1::KIF16B1-400 fusion protein together with endogenous microtubule cytoskeleton structures.

### 2. Co-localization of the synthetic organelle and the target product

Organelles generally have selective permeability and properties for sorting biomolecules. Therefore, the composition and physicochemical properties of biomolecules within the organelle compartments are significantly different from those of the components outside the organelles (such as the cytosol), thereby enabling organelles to perform specific functions.

In the present disclosure, the target product may be localized in the synthetic organelle by non-specific and/or specific binding. Preferably, a specific receptor/ligand pair is used, and the receptor and ligand can be selected as needed. For example, in one embodiment, the recruited ligand is tandem-dimer MS2 coat protein (tdMCP), and the receptor is MS2 hairpin RNA aptamer. Other ligand/receptor pairs may be used as needed, for example:
- Complementary nucleic acid molecules with any sequences;
- RNA-binding protein and corresponding RNA, for example:
   ∘ Capsid proteins of RNA phage and translation operator RNA hairpin, the RNA phage including MS2, PP7, Qbeta, etc.;
   ∘ Protein N and boxB RNA hairpin of DNA phage, the DNA phage including λ, 21, P22, etc.;
   ∘ CRISPR Cas protein and CRISPR RNA hairpin, for example Csy4 (Cas6f) derived from *Pseudomonas aeruginosa* PA14 strain and PA14 RNA hairpin;
   ∘ Other binding pairs, such as PUF-8 and 5'-UGUANAUA-3'; FBF-2 and 5'-UGURNNAUA-3'; protein A and protein A-binding aptamer; Archaeal RNA-binding protein L7Ae and RNA C/D box, etc.;
- DNA-binding protein and corresponding DNA, for example: TetR and tetO operator; cI repressor protein and cI operator; Gal4 and corresponding DNA; Zif268 DNA-binding domain and GATGCTGCA sequence; Gli-1 DNA-binding domain and GACCACCCAAGACGA sequence; LexA DNA-binding domain and CTGTATATATATACAG sequence; Transcription activator-like (TAL) domains and corresponding DNA;
- Homodimers or multimers, for example: reversible green fluorescent protein Dronpa;
- Split proteins, for example: split fluorescent proteins, such as split green fluorescent protein GFP1-10 and GFP11; split β-galactosidase; split T7 polymerase; split esterase; split TEV protease; split dihydrofolate reductase; split β-lactamase; split firefly luciferase; split thymidine kinase; split chorismate mutase; split CRISPR-Cas9; split horseradish peroxidase, etc.;
- Antigen and specific antibody, for example: His tag and anti-his antibody; flag tag and anti-flag antibody; HA tag and anti-HA antibody; Myc tag and anti-Myc antibody; GST protein and anti-GST antibody;
- Other protein-protein binding pairs, for example: SYNZIP peptide pair; SZ17 and SZ18 pair; Fos and Jun pair; *Arabidopsis thaliana* phytochrome B (PhyB) and PIF3 protein or PIF6 protein.

Certainly, receptor and ligand do not have to be a bimolecular pair, but may also form a complex multicomponent complex, such as the quaternary complex of CRISPR-Cas9 protein, crRNA, tracrRNA, and crRNA-targeted DNA; and the ternary complex of FKBP protein, FRB protein, and rapamycin.

In the synthetic organelle, receptors can be covalently connected to components that construct the synthetic organelle structure. For example, in one embodiment, the receptor MS2 hairpin RNA aptamer and the CAG repeat nucleotides that construct the synthetic organelle structure constitute a fusion RNA molecule. For example, in another embodiment, the receptor dCsy4 and protein A that constructs the synthetic organelle structure constitute a fusion protein molecule. Certainly, receptors can also be assembled in synthetic organelles through non-covalent intermolecular interactions. For example, CAG repeat nucleotides that construct the synthetic organelle structure are connected with MS2 hairpin RNA aptamer. The MS2 hairpin RNA aptamer non-covalently binds tdMCP-His tag. His tag non-covalently binds anti-His antibody and PhyB fusion protein. Then PhyB serves as a receptor, and the synthetic organelle can specifically bind proteins connected with the ligand PIF3 or PIF6. The synthetic organelle can have one or more receptors with different properties.

The linkage between a ligand and an expression product can be covalent. For example, in one embodiment, the ligand tdMCP and the target product green fluorescent protein constitute a fusion protein. Certainly, ligands can also be attached to target products through non-covalent intermolecular interactions. For example, through intermolecular interactions, the target product Dronpa145N mutant can form a heterodimer with the ligand tdMCP-Dronpa145K. The latter can be specifically recruited by synthetic organelles with MS2 hairpin RNA. The target product can also be connected with one or more ligands.

### 3. Gene expression products

The gene expression product involved in the present disclosure may be an RNA, a protein, a nucleoprotein complex, or a small molecule synthesized under the encoding of a heterologous DNA. Preferably, it is a recombinant protein, such as superfolder green fluorescent protein, restriction endonuclease DpnI, restriction endonuclease BsaI, human proinsulin, ROR-alpha, red fluorescent protein mKate2, T4 DNA ligase, artificially designed polypeptides; or recombinant RNA, such as *E. coli* 16S rRNA; or DNA, such as recombinant plasmids, single-stranded DNA; or complexes formed by recombinant macromolecules and endogenous macromolecules, such as 30S ribosomal subunits assembled from recombinantly expressed 16S rRNA and endogenous ribosomal proteins; or other cell structures different from the synthetic organelle, such as cytoskeleton and other organelles.

### 4. DNA constructs

According to the present disclosure, an artificially designed DNA construct is introduced into a host. The DNA construct is a polynucleotide containing multiple coding genes, non-coding DNA, regulatory elements, etc.

The DNA construct can guide the host to construct the synthetic organelle. For example, in one embodiment, the synthetic organelle is constructed from RNA molecules of CAG triplet nucleotide repeats, and the DNA construct contains a nucleotide sequence expressing this RNA molecule, including a sequence of CAG triplet nucleotide repeats, a transcription terminator, and a promoter regulating the inducible expression.

The DNA construct according to the present disclosure may be one or more plasmids, cosmids, or artificial chromosomes, may be integrated into single or multiple sites in the host genome, or may be a combination of the above schemes. Preferably, the DNA construct is a plasmid composed of the nucleotide sequence guiding the host to construct the synthetic organelle and to produce the target product with a plasmid vector. The upstream promoter may be a constitutive promoter or an inducible promoter. The plasmid vector may be a commercially available vector, such as pACYC184, pACYC177, pET28a(+), pET28b(+), pET-5a(+), pET43.1a, pET-37b(+), pCDFDuet-1, pCOLADuet-1, pRSFDuet-1, pETDuet-1, pUC57, pUC19, pBAD, pBluescript II SK(+), pTrcHis C, pTrcHis A, pTrcHis2C, pBV221, pQE-70, pCold III, pRSET-CFP, pRSET-BFP, pGFPuv, pKD46, pKD4, pTYB1, PinPoint Xa-2, pTWIN1, or pRSET C, or modified structures of a commercially available vector, or non-commercial vectors, such as pSB1C3, pSB3C5, pSB4C5, and pSB4K5, or other artificially designed nucleotide sequences capable of stable replication in a host cell.

### 5. Expression systems

The above DNA construct is introduced into a host for expression, guiding the host to construct the synthetic organelle, and/or regulating the host to produce the target product. The expression system may be a cell-free expression system and/or a cell expression system. The cell expression system includes prokaryotic cells and eukaryotic cells. Eukaryotic cells include, for example, yeast, fungal cells, animal cells, or plant cells, or prokaryotic cells include for example *E. coli*, *Bacillus subtilis*, lactic acid bacteria, bifidobacteria, and streptomycetes. Prokaryotic expression systems have the advantages of rapid cell reproduction, low culturing cost, and high yields, and are preferred, with *E. coli* being more preferred.

### 6. Method for decoupling gene expression product from host's internal environment

The present disclosure provides a method for decoupling the gene expression product from the host's internal environment by using a synthetic organelle, the method comprising the following steps.
a) constructing a synthetic organelle in a host.
b) localizing the gene expression product in the synthetic organelle so that the synthetic organelle is co-expressed with the gene expression product.

More specifically, step a) refers to introducing a DNA construct into the host and guiding the host to construct the synthetic organelle, and step b) refers to localizing the gene expression products in the synthetic organelle so that the synthetic organelle and the gene expression products are co-expressed. Preferably, steps a) and b) constitute a process in a single host cell in which the synthetic organelle is constructed, the target product is biosynthesized, and the target product is specifically enriched by the synthetic organelle.

### 7. Uses

The uses of the present disclosure are mainly in two aspects. One aspect is improvement of the influence of expression products on the host, including improving physiological activity and tolerance, the biomass under the same conditions, the gene expression rate, and the cell growth rate of the host. In one embodiment, for example, use of a synthetic organelle can eliminate the general stress on the host caused by expression of the sfGFP gene. In another embodiment, use of a synthetic organelle can avoid expression of an N-terminally linked toxic protein.

The other aspect is to avoid the influence of the host's internal environment on the expression product, including promoting maturation of the gene expression product, preferably promoting folding of biomacromolecules; post-expression modifications such as disulfide-bond formation, glycosylation, methylation, and acetylation; cleavage and ligation such as zymogen cleavage activation, RNA splicing, intein-mediated protein cleavage, and self-cyclization; and non-covalent assembly such as dimerization and polymerization. In one embodiment, for example, the synthetic organelle provides an environment for disulfide-bond formation, thereby reducing protein misfolding. In another embodiment, use of a synthetic organelle promotes folding of ROR-alpha and produces a soluble protein.

Accordingly, the present disclosure provides a universal solution that can simultaneously achieve a high expression level, low stress/toxicity, correct folding, and post-expression modifications.

### Examples

### Example 1: GFP

### System Design

In this example, superfolder green fluorescent protein (sfGFP) was expressed, which is a widely used fluorescent protein as a tag and generally considered non-cytotoxic. In this example, the molecules constituting the synthetic organelle were RNA fragments of 46 CAG repeats. This RNA can undergo liquid-liquid phase separation through interactions. This example used tandem-dimer MS2 coat protein (tdMCP) as the ligand and MS2 RNA hairpin as the receptor to enrich superfolder green fluorescent protein (sfGFP). tdMCP was connected to both the N-terminus and the C-terminus of sfGFP through protein-fusion linkers. The expression vector used in this example was pET28a, and the corresponding selection marker was kanamycin resistance.

### Experimental Procedure

pGFP and prCAG-MS2 were co-transformed into *E. coli* BL21(DE3) competent cells (TransGen). For each protein, three single colonies were picked and cultured at 37°C and 220 rpm for 16 hours. The overnight cultures were diluted 200 fold and cultured for 3 hours under two conditions separately: in a 96-well plate and in a 100 mL shake flask. Then the inducers isopropyl β-D-1-thiogalactopyranoside (IPTG) (Sangon Biotech) and anhydrotetracycline hydrochloride (aTc) (J&K Scientific) were added to 0.5 mM and 150 ng/mL, respectively, to induce expression during overnight culturing for about 12 hours. In the 96-well plate, growth curves were measured overnight by reading on a Tecan Spark plate reader during culturing.

### Results

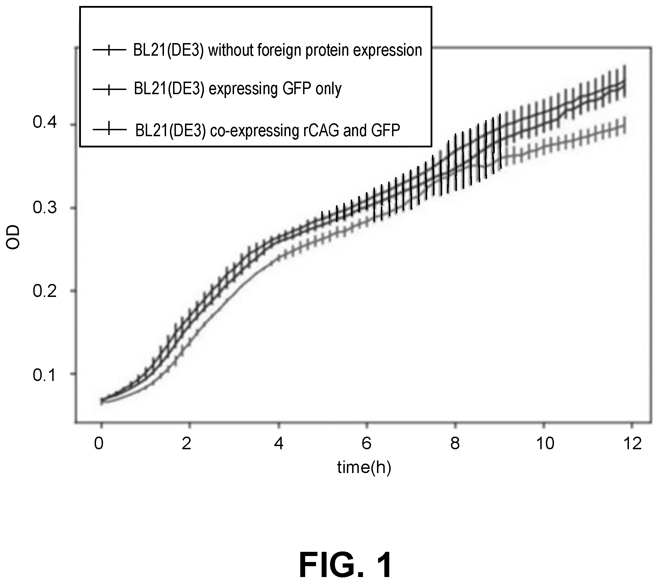

As shown in Figure 1, co-expression of the synthetic organelle significantly improved growth of the host cells, indicating that use of the synthetic organelle can eliminate the general stress on the host caused by gene expression.

### Sequence Details

The size of sfGFP is 26 kDa, and the protein sequence thereof is shown in SEQ ID NO: 1 below:

MSKGEELFTGVVPILVELDGDVNGHKFSVRGEGEGDATNGKLTLKFICTTGKLPVPWPTLVTTLT
YGVQCFSRYPDHMKRHDFFKSAMPEGYVQERTISFKDDGTYKTRAEVKFEGDTLVNRIELKGID
FKEDGNILGHKLEYNFNSHNVYITADKQKNGIKANFKIRHNVEDGSVQLADHYQQNTPIGDGPV
LLPDNHYLSTQSVLSKDPNEKRDHMVLLEFVTAAGITHG (SEQ ID NO:1)

The RNA sequence of rCAG-MS2 is shown in SEQ ID NO: 2 below:

cagcagcagcagcagcagcagcagcagcagcagcagcagcagcagcagcagcagcagcagcagcagcagcagcagcagcagcagcagca
gcagcagcagcagcagcagcagcagcagcagcagcagcagcagcagagacggagcucgucgacgcggccgcaagcuugguaccgagcu
cggauccuaagguaccuaauugccuagaaaacaugaggaucacccaugucugcaggucgacucuagaaaacaugaggaucacccaugucu
gcaguauucccggguucauuagauccuaagguaccuaauugccuagaaaacaugaggaucacccaugucugcaggucgacucuagaaaac
augaggaucacccaugucugcaguauucccggguucauuagauccuaagguaccuaauugccuagaaaacaugaggaucacccaugucug
caggucgacucuagaaaacaugaggaucacccaugucugcaguauucccggguucauuagauccuaagguaccuaauugccuagaaaaca
ugaggaucacccaugucugcaggucgacucuagaaaacaugaggaucacccaugucugcaguauucccggguucauuagauccuaaggua

ccuaauugccuagaaaacaugaggaucacccaugucugcaggucgacucuagaaaacaugaggaucacccaugucugcaguauucccggg
uucauuagauccuaagguaccuaauugccuagaaaacaugaggaucacccaugucugcaggucgacucuagaaaacaugaggaucaccca
ugu (SEQ ID NO:2)

The protein sequence of tdMCP is shown in SEQ ID NO: 3 below:

MASNFTQFVLVDNGGTGDVTVAPSNFANGVAEWISSNSRSQAYKVTCSVRQSSAQNRKYTIKV
EVPKVATQTVGGVELPVAAWRSYLNMELTIPIFATNSDCELIVKAMQGLLKDGNPIPSAIAANSG
IYANFTQFVLVDNGGTGDVTVAPSNFANGVAEWISSNSRSQAYKVTCSVRQSSAQNRKYTIKVE
VPKVATQTVGGVELPVAAWRSYLNMELTIPIFATNSDCELIVKAMQGLLKDGNPIPSAIAANSGI
Y(SEQ ID NO:3)

### Example 2: DpnI

### System Design

In this example, the restriction endonuclease DpnI was expressed, which cleaves the DNA strand containing 6mA-methylated GATC and is lethal to a common *E. coli* expression host. In this example, the molecules constituting the synthetic organelle were RNA fragments of 46 CAG repeats. This RNA can undergo liquid-liquid phase separation through interactions. This example used tandem-dimer MS2 coat protein (tdMCP) as the ligand and MS2 RNA hairpin as the receptor to enrich DpnI which identifies and cleaves the DNA strand containing 6mA-methylated GATC. tdMCP was connected to the N-terminus of DpnI through a protein fusion linker, and the fusion linker used a SNAC-tag capable of Ni²⁺-induced self-cleavage. The expression vector used in this example was pET28a, and the corresponding selection marker was kanamycin resistance.

### Experimental Procedure

pET28a-DpnI and prCAG-MS2 were co-transformed into *E. coli* BL21(DE3) competent cells (TransGen). For each protein, three single colonies were picked and cultured at 37°C and 220 rpm for 16 hours. The overnight cultures were diluted 200 fold and cultured for 3 hours, and then the inducers isopropyl β-D-1-thiogalactopyranoside (IPTG) (Sangon Biotech) and anhydrotetracycline hydrochloride (aTc) (J&K Scientific) were added to 0.5 mM and 150 ng/mL, respectively, to induce expression during overnight culturing for about 12 hours. The culture was centrifuged and the supernatant was removed. A lysis buffer (One-Step Bacterial Lysis Kit, Sangon Biotech) was added to the pellet, followed by mixing by pipetting, a reaction at room temperature for 30 min, centrifuging, and removal of the supernatant to recover the pellet. A Ni²⁺-containing cleavage eluent was added to the pellet obtained above and cleavage was conducted at room temperature on a rotating mixer. After cleavage, the sample was centrifuged and the supernatant containing the target protein was recovered. SDS-PAGE (SurePAGE^{™}, GenScript) was used to verify solubility of the target protein and assess the expression level of the recovered protein.

### Results

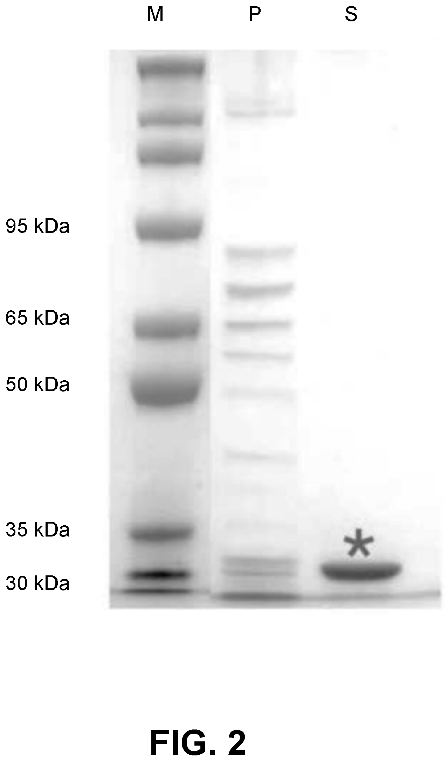

As shown in Figure 2, DpnI was expressed at a high level in a soluble form in the *E. coli* host, indicating that the synthetic organelle sufficiently recruited DpnI expressed in the cytoplasmic matrix, allowing the *E. coli* host to continue effective proliferating to a sufficient biomass even under DpnI overexpression, thereby increasing the total DpnI yield. This demonstrates that co-expression of the synthetic organelle and an N-terminally linked toxic protein can eliminate the toxicity of the toxic protein to the host.

### Sequence Details

The protein sequence of DpnI is shown in SEQ ID NO: 4 below:

MELHFNLELVETYKSNSQKARILTEDWVYRQSYCPNCGNNPLNHFENNRPVADFYCNHCSEEFE
LKSKKGNFSSTINDGAYATMMKRVQADNNPNFFFLTYTKNFEVNNFLVLPKQFVTPKSIIQRKPL
APTARRAGWIGCNIDLSQVPSKGRIFLVQDGQVRDPEKVTKEFKQGLFLRKSSLSSRGWTIEILNC
IDKIEGSEFTLEDMYRFESDLKNIFVKNNHIKEKIRQQLQILRDKEIIEFKGRGKYRKL (SEQ ID
NO:4)

The protein sequence of SNAC-tag is shown in SEQ ID NO:5 below:
GSHHW (SEQ ID NO:5)

### Example 3: BsaI

### System Design

In this example, the restriction endonuclease BsaI was expressed, which specifically recognizes GGTCTC and cleaves DNA strands and is toxic to a common *E. coli* host. In this example, the molecules constituting the synthetic organelle were RNA fragments of 46 CAG repeats. This RNA can undergo liquid-liquid phase separation through interactions. This example used tandem-dimer MS2 coat protein (tdMCP) as the ligand and MS2 RNA hairpin as the receptor to enrich BsaI which identifies and cleaves the DNA strands. tdMCP was connected to the N-terminus of BsaI through a protein fusion linker, and the fusion linker used a SNAC-tag capable of Ni²⁺-induced self-cleavage. The expression vector used in this example was pET28a, and the corresponding selection marker was kanamycin resistance.

### Experimental Procedure

pET28a-tdMCP-BsaI and prCAG-MS2 were co-transformed into *E. coli* BL21(DE3) competent cells (TransGen). For each protein, three single colonies were picked and cultured at 37°C and 220 rpm for 16 hours. The overnight cultures were diluted 200 fold and cultured for 3 hours, and then the inducers isopropyl β-D-1-thiogalactopyranoside (IPTG) (Sangon Biotech) and anhydrotetracycline hydrochloride (aTc) (J&K Scientific) were added to 0.5 mM and 150 ng/mL, respectively, to induce expression during overnight culturing for about 12 hours. The culture was centrifuged and the supernatant was removed. A lysis buffer (One-Step Bacterial Lysis Kit, Sangon Biotech) was added to the pellet, followed by mixing by pipetting, a reaction at room temperature for 30 min, centrifuging, and removal of the supernatant to recover the pellet. A Ni²⁺-containing cleavage eluent was added to the pellet obtained above and cleavage was conducted at room temperature on a rotating mixer. After cleavage, the sample was centrifuged and the supernatant containing the target protein was recovered. SDS-PAGE (SurePAGE^{™}, GenScript) was used to verify solubility of the target protein and assess the expression level of the recovered protein.

### Results

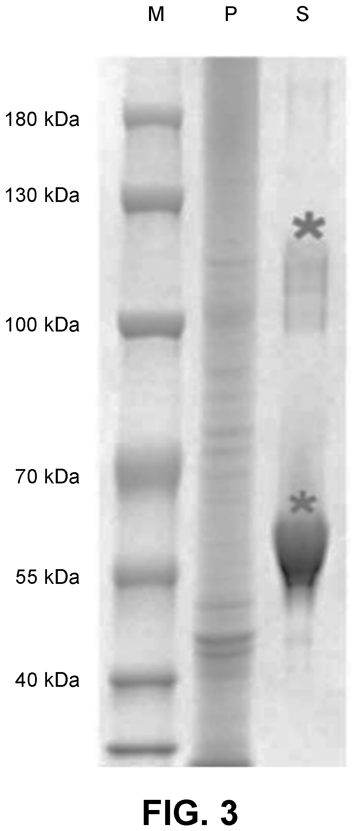

As shown in Figure 3, BsaI was expressed at a high level in a soluble form in the *E. coli* host, indicating that the synthetic organelle sufficiently recruited BsaI expressed in the cytoplasmic matrix, allowing the *E. coli* host to continue effective proliferating to a sufficient biomass even under BsaI overexpression, thereby increasing the total BsaI yield. This demonstrates that co-expression of the synthetic organelle and an N-terminally linked toxic protein can eliminate the toxicity of the toxic protein to the host.

### Sequence Details

The protein sequence of BsaI is shown in SEQ ID NO: 6 below.

MGKKAEYGQGHPIFLEYAEQIIQHKEYQGMPDLRYPDGRIQWEAPSNRKSGIFKDTNIKRRKWW
EQKAISIGIDPSSNQWISKTAKLIHPTMRKPCKKCGRIMDLRYSYPTKNLIKRIRKLPYVDESFEIDS
LEHILKLIKRLVLQYGDKVYDDLPKLLTCKAVKNIPRLGNDLDTWLNWIDSVYIPSEPSMLSPGA
MANPPDRLDGFHSLNECCRSHADRGRWEKNLRSYTTDRRAFEYWVDGDWVAADKLMGLIRTN
EQIKKETCLNDNHPGPCSADHIGPISLGFVHRPEFQLLCNSCNSAKNNRMTFSDVQHLINAENNG
EEVASWYCKHIWDLRKHDVKNNENALRLSKILRDNRHTAMFILSELLKDNHYLFLSTFLGLQYA
ERSVSFSNIKIENHIITGQISEQPRDTKYTEEQKARRMRIGFEALKSYIEKENRNALLVINDKIIDKIN
EIKNILQDIPDEYKLLNEKISEQFNSEEVSDELLRDLVTHLPTKESEPANFKLARKYLQEIMEIVGD
ELSKMWEDERYVRQTFADLD (SEQ ID NO:6).

### Example 4: Proinsulin

### Experimental Design

In this example, human proinsulin was expressed, which contains two disulfide bonds, and would form insoluble inclusion bodies when expressed in common *E. coli* hosts because of failure to form disulfide bonds. In this example, the molecules constituting the synthetic organelle were RNA fragments of 46 CAG repeats. This RNA can undergo liquid-liquid phase separation through interactions, and maintains an oxidative environment by isolating it from the enzymes that normally provide a reducing potential for the *E. coli* cytoplasmic matrix. This example used tandem-dimer MS2 coat protein (tdMCP) as the ligand and MS2 RNA hairpin as the receptor to enrich proinsulin. tdMCP was connected to the N-terminus of proinsulin through a protein fusion linker, and the fusion linker used a SNAC-tag capable of Ni²⁺-induced self-cleavage. The expression vector used in this example was pET28a, and the corresponding selection marker was kanamycin resistance.

### Experimental Procedure

pET28a-tdMCP-proinsulin and prCAG-MS2 were co-transformed into *E. coli* BL21(DE3) competent cells (TransGen). For each protein, three single colonies were picked and cultured at 37°C and 220 rpm for 16 hours. The overnight cultures were diluted 200 fold and cultured for 3 hours, and then the inducers isopropyl β-D-1-thiogalactopyranoside (IPTG) (Sangon Biotech) and anhydrotetracycline hydrochloride (aTc) (J&K Scientific) were added to 0.5 mM and 150 ng/mL, respectively, to induce expression during overnight culturing for about 12 hours. The culture was centrifuged and the supernatant was removed. A lysis buffer (One-Step Bacterial Lysis Kit, Sangon Biotech) was added to the pellet, followed by mixing by pipetting, a reaction at room temperature for 30 min, centrifuging, and removal of the supernatant to recover the pellet. A Ni²⁺-containing cleavage eluent was added to the pellet obtained above and cleavage was conducted at room temperature on a rotating mixer. After cleavage, the sample was centrifuged and the supernatant containing the target protein was recovered. SDS-PAGE (SurePAGE^{™}, GenScript) was used to verify solubility of the target protein and assess the expression level of the recovered protein.

### Results

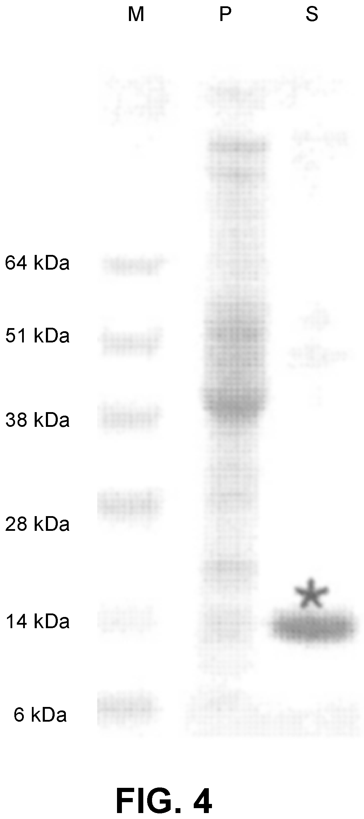

As shown in Figure 4, human proinsulin was expressed in a soluble form in the *E. coli* host, indicating that the synthetic organelle provided an environment allowing disulfide-bond formation, reduced protein misfolding, and promoted folding and disulfide-bond formation of human proinsulin, thereby yielding soluble human proinsulin.

### Sequence Details

The protein sequence of human proinsulin is shown in SEQ ID NO: 7 below.

FVNQHLCGSHLVEALYLVCGERGFFYTPKTRREAEDLQVGQVELGGGPGAGSLQPLALEGSLQK
RGIVEQCCTSICSLYQLENYCN (SEQ ID NO:7)

### Example 5: ROR-alpha

### Experimental Design

In this example, ROR-alpha was expressed, which is a human nuclear receptor that does not require a special modification but is prone to misfolding, and would form insoluble inclusion bodies in common *E. coli* hosts. In this example, the molecules constituting the synthetic organelle were RNA fragments of 46 CAG repeats. This RNA can undergo liquid-liquid phase separation through interactions. This example used tandem-dimer MS2 coat protein (tdMCP) as the ligand and MS2 RNA hairpin as the receptor to enrich ROR-alpha. tdMCP was connected to the N-terminus of ROR-alpha through a protein fusion linker. The expression vector used in this example was pET28a, and the corresponding selection marker was kanamycin resistance.

### Experimental Procedure

pET28a-tdMCP-ROR-alpha and prCAG-MS2 were co-transformed into *E. coli* BL21(DE3) competent cells (TransGen). For each protein, three single colonies were picked and cultured at 37°C and 220 rpm for 16 hours. The overnight cultures were diluted 200 fold and cultured for 3 hours, and then the inducers isopropyl β-D-1-thiogalactopyranoside (IPTG) (Sangon Biotech) and anhydrotetracycline hydrochloride (aTc) (J&K Scientific) were added to 0.5 mM and 150 ng/mL, respectively, to induce expression during overnight culturing for about 12 hours. The culture was centrifuged and the supernatant was removed. A lysis buffer (One-Step Bacterial Lysis Kit, Sangon Biotech) was added to the pellet, followed by mixing by pipetting, a reaction at room temperature for 30 min, centrifuging, and removal of the supernatant to recover the pellet. A Ni²⁺-containing cleavage eluent was added to the pellet obtained above and cleavage was conducted at room temperature on a rotating mixer. After cleavage, the sample was centrifuged and the supernatant containing the target protein was recovered. SDS-PAGE (SurePAGE^{™}, GenScript) was used to verify solubility of the target protein and assess the expression level of the recovered protein.

### Results

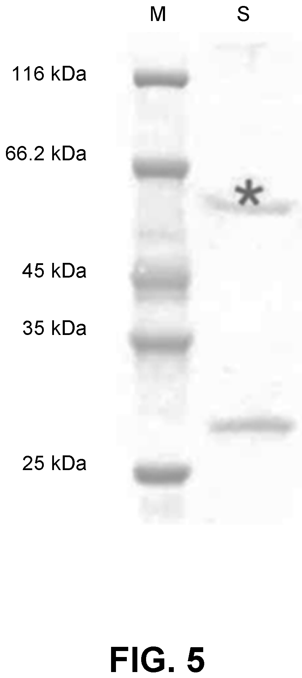

As shown in Figure 5, human ROR-alpha was expressed in a soluble form in the *E. coli* host, indicating that the synthetic organelle reduced protein misfolding, promoted folding of ROR-alpha, and yielded soluble protein.

### Sequence Details

The protein sequence of human ROR-alpha is shown in SEQ ID NO: 8 below.

AELEHLAQNISKSHLETCQYLREELQQITWQTFLQEEIENYQNKQREVMWQLCAIKITEAIQYVV
EFAKRIDGFMELCQNDQIVLLKAGSLEVVFIRMCRAFDSQNNTVYFDGKYASPDVFKSLGCEDFI

SFVFEFGKSLCSMHLTEDEIALFSAFVLMSADRSWLQEKVKIEKLQQKIQLALQHVLQKNHRED
GILTKLICKVSTLRALCGRHTEKLMAFKAIYPDIVRLHFPPLYKELFTS (SEQ ID NO:8).

## Claims

1. A synthetic organelle-assisted gene expression method, comprising the following steps:
a. constructing a synthetic organelle in a host;
b. co-expressing the synthetic organelle and a gene expression product, wherein the gene expression product is capable of being localized in the synthetic organelle.

2. The method according to claim 1, wherein the host includes a cell-free expression system and a cellular host, preferably a prokaryotic cellular host, more preferably *Escherichia coli*.

3. The method according to claim 1, wherein the synthetic organelle is an artificially designed compartmentalized structure,
preferably, wherein the synthetic organelle is a membraneless organelle, which is a biomolecular condensate formed in the host by polypeptides and/or proteins and/or DNAs and/or RNAs and/or nucleoprotein complexes,
preferably, wherein the biomacromolecules used to construct the synthetic organelle are selected from the group consisting of: nucleotide sequences with 10-1,000 consecutive repeats of repeat units, such as CAG triplets, CCUG, GGGAA, and GGGGCC; amino acid sequences with 3-500 consecutive repeats, such as repeats of resilin-like peptides (RLP); intrinsically disordered regions, such as FUS protein and ParB protein; polymerization of multivalent biomacromolecules, such as PTB protein with UCUCU sequence repeats; artificially designed binary protein two-dimensional structures; and artificially designed binary RNA two-dimensional structures.

4. The method according to claim 1, wherein the gene expression product is an RNA, a protein, a nucleoprotein complex, or a small molecule synthesized by a heterologous encoding DNA, preferably a recombinant protein, such as superfolder green fluorescent protein, restriction endonuclease DpnI, restriction endonuclease BsaI, human proinsulin, or ROR-alpha,
preferably, wherein the gene expression product matures in the synthetic organelle.

5. The method according to any one of claims 1 to 4, wherein in step b, the gene expression product is localized in the synthetic organelle by non-specific and/or specific binding,
preferably, wherein a specific receptor/ligand pair is used, the synthetic organelle has the receptor, and the gene expression product is connected with the ligand, such that the synthetic organelle can specifically bind the gene expression product;
preferably, wherein the receptor/ligand pair includes complementary nucleic acid molecule pairs; capsid proteins of RNA phages such as MS2, PP7, and Qbeta, and translation operator RNA hairpins; two segments of a split protein, such as split green fluorescent protein, alpha and omega fragments of beta-galactosidase, and split T7 RNA polymerase.

6. A DNA construct comprising nucleotide sequence 1 that guides a host to construct the synthetic organelle defined in the method according to any one of claims 1 to 5, and/or nucleotide sequence 2 that guides and regulates the generation of the gene expression product defined in the method according to any one of claims 1 to 5,
preferably, wherein the DNA construct is composed of nucleotide sequences 1 and 2 and a plasmid vector, said plasmid vector being for example pACYC184, pACYC177, pET28a(+), pET28b(+), pET-5a(+), pET43.1a, pET-37b(+), pCDFDuet-1, pCOLADuet-1, pRSFDuet-1, pETDuet-1, pUC57, pUC19, pBAD, pBluescript II SK(+), pTrcHis C, pTrcHis A, pTrcHis2C, pBV221, pQE-70, pCold III, pRSET-CFP, pRSET-BFP, pGFPuv, pKD46, pKD4, pTYB1, PinPoint Xa-2, pTWIN1, pRSET C, pSB1C3, pSB3C5, pSB4C5, or pSB4K5,
preferably, wherein the upstream promoter is a constitutive promoter or an inducible promoter.

7. An expression system comprising the DNA construct according to claim 6,
preferably, wherein the expression system is a system that simultaneously constructs the synthetic organelle and generates the gene expression product in a prokaryotic cell,
more preferably, wherein the expression system is an *E. coli* expression system.

8. Use of a synthetic organelle in eliminating the stress and/or toxicity caused by gene expression to a host and/or in decoupling a gene expression product from a host's internal environment, the use comprising co-expressing the synthetic organelle and the gene expression product in the host, wherein the gene expression product is capable of being localized in the synthetic organelle.

9. The use according to claim 8, wherein the use comprises improving the physiological activity and tolerance, the biomass under the same conditions, the gene expression rate, and/or the cell growth rate of the host.

10. The method according to claim 4 or the use according to claim 8, wherein the gene expression product matures in the synthetic organelle,
preferably, wherein the maturation comprises one or more of: folding of biomacromolecules; post-expression modifications, such as disulfide-bond formation, glycosylation, methylation, and acetylation; cleavage and ligation, such as zymogen cleavage activation, RNA splicing, intein-mediated protein cleavage, and self-cyclization; and non-covalent assembly, such as dimerization and polymerization.

## Amended claims

### Amended claims under Art. 19.1 PCT

1. [Amended] Use of a synthetic organelle in optimizing gene expression in a host, wherein the gene expression is optimized by a method of decoupling the gene expression products from the host's internal environment by using the synthetic organelle, said method comprising:
a. in a host, constructing a synthetic organelle, and expressing a target gene; and
b. in the host, enabling one or more of the gene expression products to be localized in the synthetic organelle.

2. [Amended] The use according to claim 1, wherein the host includes a cell-free expression system and a cellular host, preferably a prokaryotic cellular host, more preferably *Escherichia coli*.

3. [Amended] The use according to claim 1, wherein the synthetic organelle is an artificially designed compartmentalized structure,
preferably, wherein the synthetic organelle is a membraneless organelle, which is a biomolecular condensate formed in the host by polypeptides and/or proteins and/or DNAs and/or RNAs and/or nucleoprotein complexes,
preferably, wherein the biomacromolecules used to construct the synthetic organelle are selected from the group consisting of: nucleotide sequences with 10-1,000 consecutive repeats of repeat units, such as CAG triplets, CCUG, GGGAA, and GGGGCC; amino acid sequences with 3-500 consecutive repeats, such as repeats of resilin-like peptides (RLP); intrinsically disordered regions, such as FUS protein and ParB protein; polymerization of multivalent biomacromolecules, such as PTB protein with UCUCU sequence repeats; artificially designed binary protein two-dimensional structures; and artificially designed binary RNA two-dimensional structures.

4. [Amended] The use according to claim 1, wherein the gene expression product is an RNA, a protein, a nucleoprotein complex, or a small molecule synthesized by a heterologous encoding DNA, preferably a recombinant protein, for example, a protein prone to aggregation or misfolding, such as superfolder green fluorescent protein, restriction endonuclease DpnI, restriction endonuclease BsaI, human proinsulin, or ROR-alpha,
preferably, wherein the gene expression product matures in the synthetic organelle.

5. [Amended] The use according to claim 1, wherein in step b, the gene expression product is localized in the synthetic organelle by non-specific and/or specific binding,
preferably, wherein a specific receptor/ligand pair is used, the synthetic organelle has the receptor, and the gene expression product is connected with the ligand, such that the synthetic organelle can specifically bind the gene expression product;
preferably, wherein the receptor/ligand pair includes complementary nucleic acid molecule pairs; capsid proteins of RNA phages such as MS2, PP7, and Qbeta, and translation operator RNA hairpins; two segments of a split protein, such as split green fluorescent protein, alpha and omega fragments of beta-galactosidase, and split T7 RNA polymerase.

6. [Amended] The use according to claim 1, wherein the use comprises eliminating or reducing the effects by the gene expression on the host,
preferably, wherein said effects include the energy and material burden imposed by the expression process, and the general stress and/or specific toxicity caused by the expression products,
preferably, wherein the use includes improving the physiological activity and tolerance, the biomass under the same conditions, the gene expression rate and/or the cell growth rate of the host.

7. [Amended] The use according to claim 1, wherein the use eliminates or reduces the effects by the host's internal environment on the gene expression products, preferably comprising promoting the maturation of the gene expression products,
preferably, wherein the gene expression product matures in the synthetic organelle,
preferably, wherein the maturation comprises one or more of: folding of biomacromolecules; post-expression modifications, such as disulfide-bond formation, glycosylation, methylation, and acetylation; cleavage and ligation, such as zymogen cleavage activation, RNA splicing, intein-mediated protein cleavage, and self-cyclization; and non-covalent assembly, such as dimerization and polymerization.

8. [Amended] A DNA construct comprising nucleotide sequence 1 that guides a host to construct the synthetic organelle defined in the use according to any one of claims 1 to 7, and/or nucleotide sequence 2 that guides and regulates the generation of the gene expression products defined in the use according to any one of claims 1 to 7,
preferably, wherein the DNA construct is composed of nucleotide sequences 1 and 2 and a plasmid vector, said plasmid vector being for example pACYC184, pACYC177, pET28a(+), pET28b(+), pET-5a(+), pET43.1a, pET-37b(+), pCDFDuet-1, pCOLADuet-1, pRSFDuet-1, pETDuet-1, pUC57, pUC19, pBAD, pBluescript II SK(+), pTrcHis C, pTrcHis A, pTrcHis2C, pBV221, pQE-70, pCold III, pRSET-CFP, pRSET-BFP, pGFPuv, pKD46, pKD4, pTYB1, PinPoint Xa-2, pTWIN1, pRSET C, pSB1C3, pSB3C5, pSB4C5, or pSB4K5,
preferably, wherein the upstream promoter is a constitutive promoter or an inducible promoter.

9. [Amended] An expression system comprising the DNA construct according to claim 8, preferably, wherein the expression system refers to a system that simultaneously constructs the synthetic organelle and generates the gene expression products in a prokaryotic cell,
more preferably, wherein the expression system is an *E. coli* expression system.

10. [Canceled]
